# EUROPÄISCHE PATENTSCHRIFT

(11) **EP 3 868 767 B1**
(45) Veröffentlichungstag und Bekanntmachung des Hinweises auf die Patenterteilung: **05.01.2022**
(21) Anmeldenummer: 20020082.2
(22) Anmeldetag: 21.02.2020
(51) Int. Cl.: C07F 7/22, C07F 7/30, A61K 6/20, A61K 6/30, A61K 6/62, A61K 6/887, C08F 2/50, C08F 4/16, C08F 4/44, C08F 4/70, C08L 33/04, C08L 33/26

(54) **LANGWELLIG ABSORBIERENDE PHOTOINITIATOREN**
LONG-WAVE ABSORBENT PHOTOINITIATORS
PHOTOINITIATEURS ABSORBANT LES ONDES LONGUES

(43) Veröffentlichungstag der Anmeldung: 25.08.2021
(73) Patentinhaber: Ivoclar Vivadent AG, 9494 Schaan (LI)
(72) Erfinder: Moszner, Norbert, 9495 Triesen (LI); Catel, Yohann, 9475 Sevelen (CH); Fässler, Pascal, 7323 Wangs (CH); Haas, Michael, 8051 Graz (AT); Radebner, Judith, 5574 Göriach (AT); Stüger, Harald, 8042 Graz (AT)
(74) Vertreter: Uexküll & Stolberg

(56) Entgegenhaltungen:
- US-B2- 8 829 067

## Beschreibung

Die vorliegende Erfindung betrifft Acylgermanium- und Acylzinn-Verbindungen, die sich als Photoinitiatoren für die Aushärtung von radikalisch polymerisierbaren Werkstoffen eignen. Die Initiatoren zeichnen sich dadurch aus, dass sie sich mit sichtbarem Licht aktivieren lassen. Sie können zur Herstellung von Adhäsiven, Beschichtungen, Zementen, Kompositen, Formteilen, wie Stäben, Platten, Scheiben oder Linsen und insbesondere zur Herstellung von Dentalwerkstoffen verwendet werden.

Für die Aushärtung von photopolyreaktionsfähigen Harzen spielt der eingesetzte Photoinitiator eine entscheidende Rolle. Bei Bestrahlung mit UV oder sichtbarem Licht absorbiert der Photoinitiator das Licht und bildet die polyreaktionsauslösenden Spezies. Im Fall der radikalischen Photopolymerisation handelt es sich dabei um freie Radikale.

Basierend auf dem chemischen Mechanismus der Radikalbildung werden die Photoinitiatoren in zwei Klassen eingeteilt. Norrish-Typ-I-Photoinitiatoren bilden beim Bestrahlen durch eine unimolekulare Bindungsspaltung freie Radikale. Norrish-Typ-II-Photoinitiatoren durchlaufen bei der Bestrahlung eine bimolekulare Reaktion, wobei der Photoinitiator im angeregten Zustand mit einem zweiten Molekül, dem sogenannten Coinitiator reagiert und durch Elektronen- und Protonentransfer die polymerisationsauslösenden Radikale bildet. Für die UV-Lichthärtung werden Typ-I- und Typ-II-Photoinitiatoren eingesetzt, für den sichtbaren Bereich kommen bisher, abgesehen von Bisacyldialkylgermanium-Verbindungen, nahezu ausschließlich Typ-II-Photoinitiatoren zum Einsatz.

Bedingt durch die geringe Wellenlänge des UV-Lichts lassen sich mit der UV-Härtung vor allem transparente Beschichtungen mit geringer Schichtstärke härten. Bei starker Einfärbung oder Pigmentierung und bei größeren Schichtstärken gelangt man an die Grenzen der UV-Härtung. In diesen Fällen ist eine vollständige Härtung mit UV-Licht nicht möglich. Werden größere Durchhärtungstiefen benötigt, wie zum Beispiel bei der Aushärtung von lichthärtenden, dentalen Füllungskompositen, wird in der Regel mit sichtbarem Licht bestrahlt. Das hierfür am häufigsten eingesetzte Photoinitiatorsystem ist die Kombination eines α-Diketons mit einem Amincoinitiator, das z.B. in GB 1 408 265 beschrieben ist.

Die US 4,457,818 und US 4,525,256 offenbaren Dentalwerkstoffe, die ein α-Diketon wie Campherchinon als Photoinitiator enthalten. Campherchinon hat ein Absorptionsmaximum bei einer Wellenlänge von 468 nm und weist daher eine starke Gelbfärbung auf, so dass mit Campherchinon/Amin initiierte Materialien nach der Aushärtung häufig einen deutlichen Gelbstich haben. Dies ist vor allem bei Werkstoffen mit hellen Weißtönen sehr nachteilig.

Die EP 1 905 415 A1 offenbart radikalisch polymerisierbare Dentalwerkstoffe, die Bisacyldialkylgermaniumverbindungen als Norrish Typ I Photoinitiatoren enthalten. Die Initiatoren lassen sich mit Blaulicht aktivieren, das im Dentalbereich häufig zur Härtung eingesetzt wird, und führen nicht zu Verfärbungen der Werkstoffe. Die konkret offenbarten Verbindungen haben Absorptionsmaxima im Bereich von 411,5 nm bis 418,5 nm.

Die EP 2 103 297 A1 offenbart radikalisch polymerisierbare Dentalwerkstoffe, die Acylgermanium-Verbindungen mit mehreren Germaniumatomen als Photoinitiatoren enthalten. Diese Initiatoren zeichnen sich durch eine geringe Zytotoxizität und eine hohe Aktivität aus und ermöglichen eine hohe Durchhärtungstiefe ohne störende Verfärbungen der Werkstoffe. Das konkret offenbarte 1,6-Bis[4-(trimethylgermiocarbonyl)phenoxy]hexan hat ein Absorptionsmaximum von 400,5 nm.

Aus der EP 3 150 641 A1 sind tetrafunktionelle Acylgermane und -stannane bekannt, die sich als Photoinitiatoren für dentale Zwecke eignen. Diese ergeben bei der Bestrahlung mit sichtbarem Licht hohe Durchhärtungstiefen und lassen sich im Vergleich zu Bisacylgermanen einfacher herstellen. Nachteilig ist, dass diese Initiatoren vergleichsweise teuer sind. Die konkret offenbarten Verbindungen weisen Absorptionsmaxima im Bereich von 288 nm bis 419 nm auf.

Der Erfindung liegt die Aufgabe zugrunde, Photoinitiatoren für die radikalische Polymerisation durch sichtbares Licht bereitzustellen, die nicht die Nachteile bekannter Initiatoren aufweisen, die sich durch eine verbesserte Härtungscharakteristik auszeichnen, und die im Vergleich zum Stand der Technik einfach herstellbar sind. Die Initiatoren sollen darüber hinaus insbesondere große Durchhärtungstiefen ermöglichen.

Diese Aufgabe erfindungsgemäß durch multifunktionelle aromatische Acylgermanium- und Acylzinn-Verbindungen gemäß der allgemeinen Formel (I) gelöst, 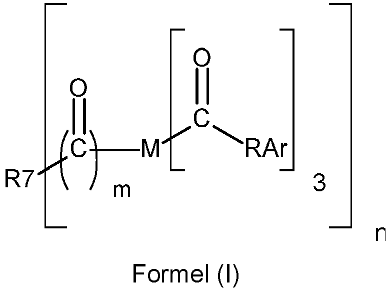 in der die Variablen die folgenden Bedeutungen haben:
M Ge oder Sn
RAr 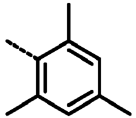
R⁷ 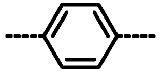
n 2
m 1
oder
M Ge
RAr 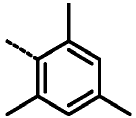
R⁷ 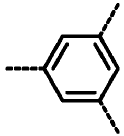
n 3
m 1
oder
M Ge
RAr 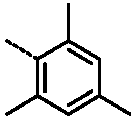
R⁷ C₂-C₈-Alkylen, vorzugsweise -C₄H₈-
n 2
m 0.

Die Gruppe R⁷ ist n-fach durch die in Klammern stehende Gruppe substituiert.

Durch die Formel (I) und die übrigen hierin gezeigten Formeln werden sämtliche stereoisomere Formen sowie Gemische verschiedener stereoisomerer Formen, wie z.B. Racemate, erfasst. Die Formel (I) erstreckt sich nur auf solche Verbindungen, die mit der chemischen Valenzlehre vereinbar sind.

Alkylen steht, auch wenn dies nicht ausdrücklich angegeben wird, für lineare und verzweigte Gruppen, wobei lineare Reste in allen Fällen bevorzugt sind.

Gemäß einer Ausführungsform der Erfindung sind solche Verbindungen der Formel (I) bevorzugt, in denen m = 1 ist. Diese Verbindungen lassen sich durch die Formel (II) darstellen:

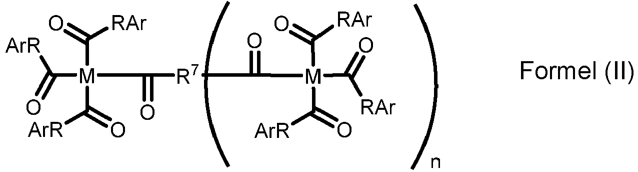

Bevorzugte Verbindungen der Formel (II) sind solche Verbindungen, bei denen die Variablen der Formel (II) die folgenden Bedeutungen haben:
M Ge oder Sn
RAr 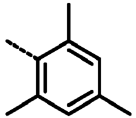
R⁷ 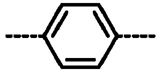
n 2.

Gemäss einer weiteren Ausführungsform der Erfindung haben die Variablen der Formel (II) vorzugsweise die folgenden Bedeutungen:
M Ge
RAr 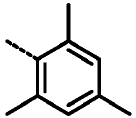
R⁷ 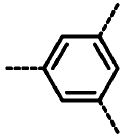
n 3.

Gemäß einer weiteren Ausführungsform der Erfindung sind solche Verbindungen der Formel (I) bevorzugt, in denen m = 0 ist. Diese Verbindungen lassen sich durch die Formel (III) darstellen:

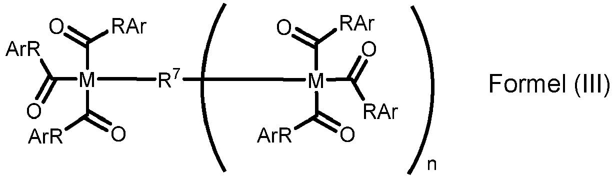

Die Variablen der Formel (III) haben die folgenden Bedeutungen:
M Ge
RAr 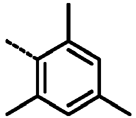
R⁷ C₂-C₈-Alkylen, besonders bevorzugt -C₄H₈-
n 2.

Die erfindungsgemäßen aromatischen Acylgermanium- oder Acylzinn-Verbindungen der allgemeinen Formel (I) sind aus dem Stand der Technik nicht bekannt. Die Synthese der Verbindungen erfolgt vorzugsweise ausgehend von den entsprechenden aromatischen Trisacylmetallenolaten (TrAME), im Falle von M = Ge ausgehend von den Trisacylgermenolaten (TrAGeE) und bei M = Sn ausgehend von den Trisacylstannenolaten (TrASnE), die durch Umsetzung der entsprechenden Tetraacylmetallverbindung, d.h. eines entsprechenden Tetraacylgermans (TAGe, M = Ge) oder Tetraacylstannans (TASn, M = Sn) mit Kalium-tert.-butylat (KO*t*Bu) zugänglich sind:

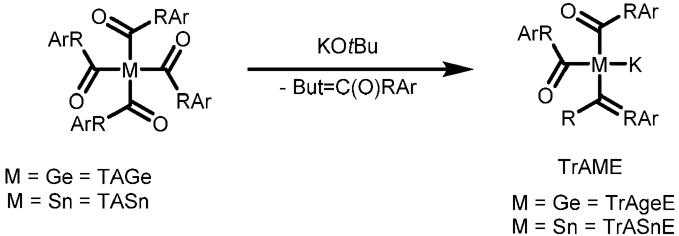

Außerdem kann die Synthese des Trisacylmetallenolates (TrAME) in einer Eintopfreaktion so erfolgen, dass man zunächst Tetrakis(trimethylsilyl)german bzw. -stannan mit KO*t*Bu zu dem entsprechenden Tris(trimethylsilyl)germanid bzw, -stannid reagieren lässt, das dann weiter mit 3 Äquivalenten eines aromatischen Säurefluorids zum Trisacylmetallenolat (TrAME) umgesetzt wird:

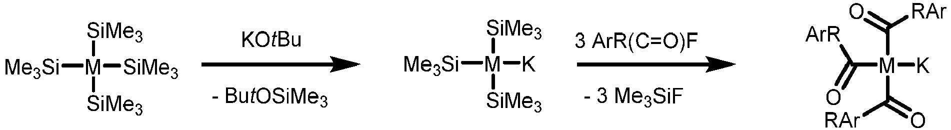

Mit den Trisacylmetallenolaten (TrAME) lassen sich dann die erfindungsgemäßen multifunktionellen aromatischen Acylgermanium- und Acylzinn-Verbindungen der allgemeinen Formel (I) herstellen.

So können die erfindungsgemäßen Octa-/Dodecaacylgermanium- und -zinn-Verbindungen der allgemeinen Formel (II) durch Umsetzung des Trisacylmetallenolates mit Di- oder Trisäurechloriden erhalten werden:

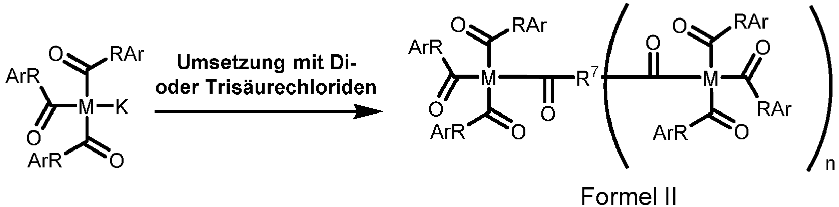

Konkretes Beispiel für Octaacylgermanium-Derivate:

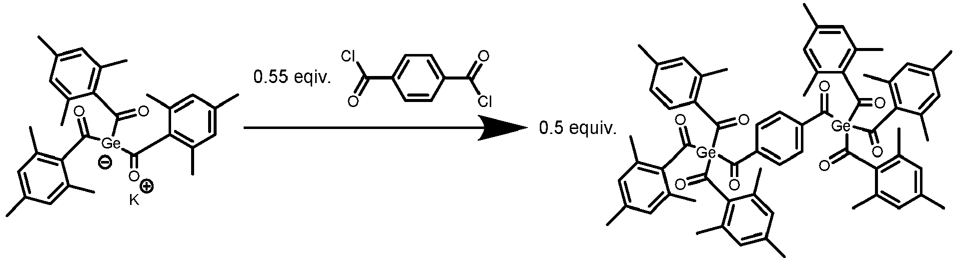

Die erfindungsgemäßen Hexaacyl-/Nonaacyl-Germanium- und -Zinn-Verbindungen der allgemeinen Formel (III) können durch Umsetzung der Trisacylmetallenolate mit α, ω- Di- oder Trihalogenalkanen hergestellt werden:

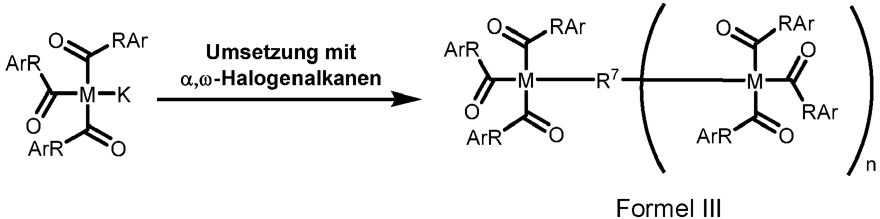

Konkretes Beispiel für ein Hexaacyldigermanium-Derivat:

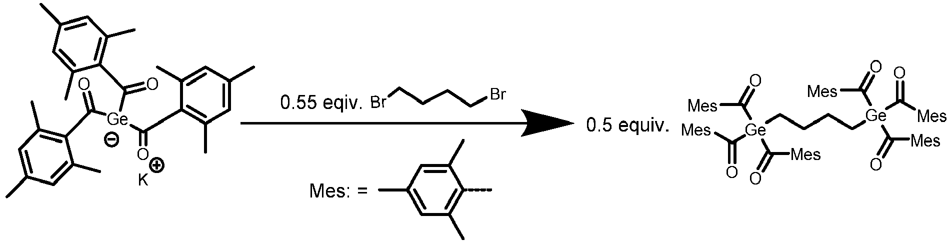

Konkrete Beispiele für besonders bevorzugte Verbindungen der Formel (I) sind:

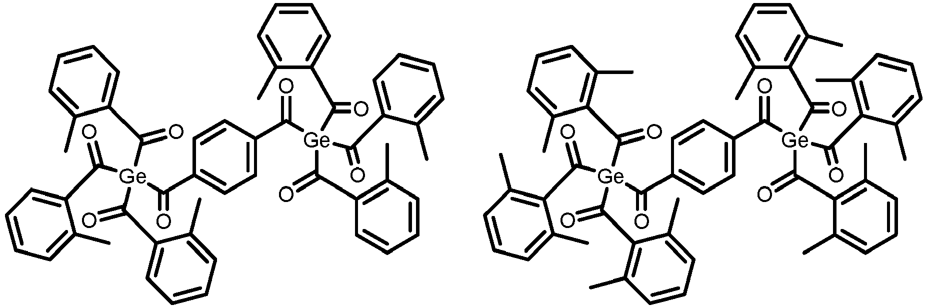

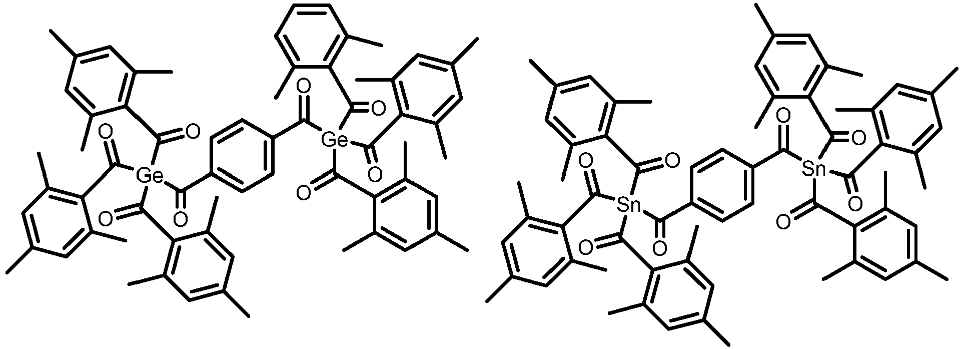

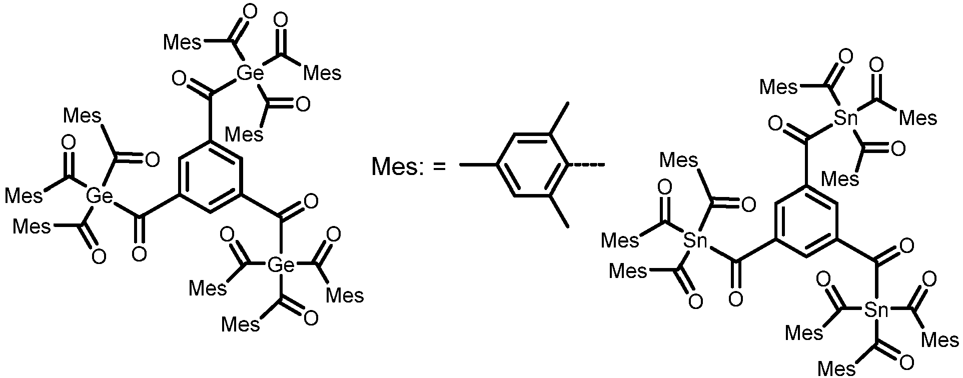

Die erfindungsgemäßen Verbindungen der allgemeinen Formel (I) weisen überraschenderweise einen Absorptionsbereich für sichtbares Licht auf, der im Vergleich zu strukturähnlichen bekannten Photoinitiatoren deutlich zu größeren Wellenlägen hin verschoben ist. Sie erlauben so die Initiierung der Polymerisation mit längerwelligem Licht und ermöglichen größere Durchhärtungstiefen. Außerdem zeichnen sich die Verbindungen der Formel (I) durch sehr hohe Extinktionskoeffizienten der Absorption im sichtbaren Bereich aus. Sie sind daher schon in geringer Konzentration als Photoinitiatoren für durch sichtbares Licht initiierte Polyreaktionen wirksam. Die Photoinitiatoren der Formel (I) haben darüber hinaus ein sehr gutes Bleachverhalten, d.h. sie werden bei der Polymerisation sehr schnell und praktisch vollständig entfärbt. Zudem sind sie synthetisch gut zugänglich.

Die erfindungsgemäßen-Verbindungen der allgemeinen Formel (I) eignen sich besonders als Photoinitiatoren für Polyreaktionen, insbesondere als Initiatoren für die Polyaddition und für die Thiol-En-Reaktion und ganz besonders für die radikalische Polymerisation. Hierzu werden sie vorzugsweise mit mindestens einem polymerisierbaren Bindemittel kombiniert. Bevorzugt sind Bindemittel auf der Basis von Monomeren, die sich durch Polyaddition polymerisieren lassen, besonders bevorzugt sind Bindemittel auf der Basis von radikalisch polymerisierbarer Monomeren. Zusammensetzungen, die mindestens eine Verbindung der Formel (I) und mindestens ein Monomer enthalten, sind ebenfalls Gegenstand dieser Erfindung.

Als radikalisch polymerisierbare Monomere eignen sich besonders mono- oder multifunktionelle (Meth)acrylate oder deren Mischung. Unter monofunktionellen (Meth)-acrylaten werden Verbindungen mit einer, unter multifunktionellen (Meth)acrylaten Verbindungen mit zwei oder mehr, vorzugsweise 2 bis 3 polymerisierbaren Gruppen verstanden.

Bevorzugte Beispiele sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, 1,4-Butandioldi(meth)-acrylat, 1,10-Decandioldimethacrylat (D₃MA), Bis(methacryloyloxymethyl)tricyclo-[5.2.1.0^{2,6}]decan (DCP), sowie Glycerindi- und Glycerintri(meth)acrylat, 1,4-Butandioldi(meth)acrylat, 1,10-Decandioldacrylat, 1,12-Dodecandioldi(meth)acrylat und Mischungen davon.

Zusammensetzungen, die mindestens ein radikalisch polymerisierbares Monomer mit 2 oder mehr, vorzugsweise 2 bis 3 radikalisch polymerisierbaren Gruppen enthalten, sind besonders bevorzugt. Mehrfach funktionelle Monomere haben vernetzende Eigenschaften.

Als radikalisch polymerisierbare Monomere lassen sich vorteilhaft auch hydrolysestabile Monomere, wie hydrolysestabile Mono(meth)acrylate, z.B. Mesitylmethacrylat oder 2-(Alkoyxymethyl)acrylsäuren, z.B. 2-(Ethoxymethyl)acrylsäure, 2-(Hydroxymethyl)acrylsäure, N-mono- oder -disubstitiuierte Acrylamide, wie z.B. N-Ethylacrylamid, N,N-Dimethacrylamid, N-(2-Hydroxyethyl)acrylamid oder N-Methyl-N-(2-hydroxyethyl)acrylamid, N-monosubstituierte Methacrylamide, wie z.B. N-Ethylmethacrylamid oder N-(2-Hydroxyethyl)methacrylamid sowie N-Vinylpyrrolidon oder Allylether einsetzen.

Bevorzugte Beispiele für hydrolysestabile Vernetzermonomere sind Urethane aus 2-(Hydroxymethyl)acrylsäure und Diisocyanten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylenoder Ethylenbisacrylamid, Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung der entsprechenden Diamine mit (Meth)acrylsäurechorid synthetisiert werden können. Bevorzugt sind bei Raumtemperatur flüssige Monomere, die als Verdünnermonomere eingesetzt werden können.

Weiterhin lassen sich als radikalisch polymerisierbare Bindemittel auch schrumpfungsarme radikalisch ringöffnend polymerisierbare Monomere wie z.B. mono- oder multifunktionelle Vinylcyclopropane bzw. bicyclische Cyclopropanderivate, vorzugsweise die in DE 196 16 183 C2 oder EP 1 413 569 A1 beschriebenen, oder cyclische Allylsulfide, vorzugsweise die in US 6,043,361 und US 6,344,556 beschriebenen, einsetzen. Diese können vorteilhaft auch in Kombination mit den voranstehend aufgeführten Di(meth)acrylat-Vernetzern verwendet werden. Bevorzugte ringöffnend polymerisierbare Monomere sind Vinylcyclopropane, wie 1,1-Di(ethoxycarbonyl)-oder 1,1-Di(methoxycarbonyl)-2-vinylcyclopropan oder die Ester der 1-Ethoxycarbonyl- oder 1-Methoxycarbonyl-2-vinylcyclopropancarbonsäure mit Ethylenglycol, 1,1,1-Trimethylolpropan, 1,4-Cyclohexandiol oder Resorcin. Bevorzugte bicyclische Cyclopropanderivate sind 2-(Bicyclo[3.1.0]hex-1-yl)acrylsäuremethyl- oder -ethylester und deren Disubstitutionsprodukte in 3-Stellung, wie (3,3-Bis(ethoxycarbonyl)bicyclo[3.1.0]hex-1-yl)acrylsäuremethyl- oder -ethylester. Bevorzugte cyclische Allylsulfide sind die Additionprodukte von 2-(Hydroxymethyl)-6-methylen-1,4-dithiepan oder 7-Hydroxy-3-methylen-1,5-dithiacylooctan mit 2,2,4-Trimethylhexamethylen-1,6-diisocyanat oder dem asymmetrischen Hexamethylendiisocyanat-Trimeren (Desmodur^{®} VP LS 2294 der Bayer AG).

Weitere bevorzugte radikalisch polymerisierbare Monomere sind Vinylester, Vinylcarbonate und Vinylcarbamate. Außerdem können als radikalisch polymerisierbare Monomere auch Styrol, Styrolderivate, Divinylbenzol, ungesättigte Polyesterharze sowie Allylverbindungen oder radikalisch polymerisierbare Polysiloxane, die aus geeigneten Methacrylsilanen, wie z.B. 3-(Methacryloyloxy)propyltrimethoxysilan, hergestellt werden können und z.B. in der DE 199 03 177 C2 beschrieben sind, eingesetzt werden. Unter Styrolderivaten werden Verbindungen verstanden, bei denen die Phenylgruppe des Styrols, nicht aber die Vinylgruppe, ein oder mehrfach durch einfache Gruppen, wie C₁-C₁₀-Alkyl, Cl, Br, OH, CH₃O, CHO, C₂H₅O, COOH oder Carbonsäureestergruppen substituiert ist.

Außerdem lassen sich als radikalisch polymerisierbare Bindemittel auch Mischungen der voranstehend genannten Monomeren mit radikalisch polymerisierbaren, säuregruppenhaltigen Monomeren verwenden, die auch als Haftmonomere bezeichnet werden. Bevorzugte säuregruppenhaltige Monomere sind polymerisationsfähige Carbonsäuren, wie Maleinsäure, Acrylsäure, Methacrylsäure, 2-(Hydroxymethyl)-acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin oder 4-Vinylbenzoesäure.

Als Haftmonomere eignen sich besonders radikalisch polymerisierbare Phosphonsäuremonomere, insbesondere Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[2-Dihydroxyphosphoryl)-ethoxymethyl]-acrylsäure-ethyl- oder 2,4,6-trimethylphenylester.

Zudem eignen sich als Haftmonomere acide polymerisationsfähige Phosphorsäureester, insbesondere 2-Methacryloyloxypropylmono- oder -dihydrogenphosphat, 2-Methacryloyloxyethylmono- oder -dihydrogenphosphat, 2-Methacryloyloxyethylphenylhydrogenphosphat, Dipentaerythritol-pentamethacryoyloxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat.

Darüber hinaus eignen sich auch polymerisationsfähige Sulfonsäuren als Haftmonomere, insbesondere Vinylsulfonsäure, 4-Vinylphenylsulfonsäure oder 3-(Methacrylamido)propylsulfonsäure.

Als durch Polyaddition härtbare Bindemittel eignen sich besonders Thiol-En-Harze, die Mischungen von mono- oder multifunktionellen Mercaptoverbindungen und dioder multifunktionellen ungesättigten Monomeren, vor allem Allyl- oder Norbonenverbindungen, enthalten.

Beispiele für mono- oder multifunktionellen Mecaptoverbindungen sind o-, m- oder p-Dimercaptobenzol und Ester der Thioglykol- oder der 3-Mercaptopropionsäure von Ethylen-, Propylen- oder Butylenglykol, Hexandiol, Glycerin, Trimethylolpropan oder Pentaerythrit.

Beispiele für di- oder multifunktionelle Allylverbindungen sind Ester von Allylalkohol mit Di- oder Tricarbonsäuren, wie Malon-, Malein-, Glutar-, Bernstein-, Adipin-, Sebacin-, Phthal-, Terephthal- oder Gallussäure sowie mono- oder trifunktionelle Allylether, wie z.B. Diallylether, α,ω-Bis[allyloxy]alkane, Resorcin- oder Hydrochinondiallylether sowie Pyrogalloltriallylether, oder andere Verbindungen wie z.B. 1,3,5-Triallyl-1,3,5-triazin-2,4,6-(1H,3H,5H)-trion, Tetraallylsilan oder Tetraallylorthosilikat.

Beispiele für di- oder multifunktionelle Norbonenverbindungen sind Diels-Alder-Additionsprodukte von Cyclopentadien oder Furan mit di- oder multifunktionellen (Meth)acrylaten, sowie Ester und Urethane von 5-Norbornen-2-methanol oder 5-Norbornen-2-ol mit Di- oder Polycarbonsäuren, wie z.B. Malon-, Malein-, Glutar-, Bernstein-, Adipin-, Sebacin-, Phthal-, Terephthal- oder Gallussäure, mit Di- oder Polyisocyanaten, wie Hexamethylendiisocyanat oder dessen cyclischem Trimer, 2,2,4-Trimethylhexamethylendiisocyanat, Toluylendiisocyanat oder Isophorondiisocyanat.

Neben Acylgermanium-Verbindungen der allgemeinen Formel (I) können die erfindungsgemäßen Zusammensetzungen vorteilhaft zusätzlich auch einen oder mehrere bekannte Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich enthalten. Geeignet sind insbesondere Kombinationen mit Norrish-Typ-I-Photoinitiatoren, vor allem Acyl- oder Bisacylphosphinoxide, wie beispielsweise die kommerziell erhältlichen Verbindungen 2,4,6-Trimethylbenzoyldiphenylphosphinoxid und Bis(2,4,6-trimethylbenzoyl)phenylphosphinoxid. Besonders geeignet sind Monoacyltrialkyl-, Diacyldialkylgermanium-, Triacylalkyl- sowie Tetraacylgermanium-Verbindungen, wie z.B. Benzoyltrimethylgermanium, Dibenzoyldiethylgermanium oder Bis(4-methoxybenzoyl)diethylgermanium sowie Tetrabenzoylgermanium. Weitere bevorzugte Mischungen sind Initiatorkombinationen, die Verbindungen der allgemeinen Formel (I) in Kombination mit aromatischen Diaryliodonium- oder Triarylsulfoniumsalzen enthalten, beispielsweise die kommerziell zugänglichen Verbindungen 4-Octyloxyphenyl-phenyliodoniumhexafluoroantimonat oder Isopropylphenyl-methylphenyl-iodoniumtetrakis-(pentafluorphenyl)borat.

Außerdem können die erfindungsgemäßen Zusammensetzungen neben den Verbindungen der allgemeinen Formel (I) zur dualen Härtung auch Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, *tert*-Butylperoctoat, *tert*-Butylperbenzoat oder Di-(*tert*-butyl)-peroxid enthalten. Zur Beschleunigung der Initiierung mittels Peroxiden lassen sich auch Kombinationen mit aromatischen Aminen einsetzen. Bevorzugte Redoxsysteme sind Kombinationen aus Benzoylperoxid mit Aminen, wie N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme bestehend aus Peroxiden und Reduktionsmitteln, wie z.B. Ascorbinsäure, Barbituraten oder Sulfinsäuren, oder Kombinationen von Hydroperoxiden mit Reduktionsmitteln und katalytischen Metallionen, wie z.B. eine Mischung von Cumolhydroperoxid, einem Thioharnstoffderivat und Kupfer(II)-acetylacetonat, für die duale Aushärtung geeignet.

Die erfindungsgemäßen Zusammensetzungen können vorteilhaft außerdem einen oder mehrere organische oder vorzugsweise anorganische Füllstoffe enthalten. Bevorzugt sind faserförmige und insbesondere partikuläre Füllstoffe.

Als faserförmige Füllstoffe sind Nanofasern, Glasfasern, Polyamidfasern und Kohlenstofffasern bevorzugt. Unter Nanofasern werden Fasern mit einer Länge von weniger als 100 nm verstanden. Faserförmige Füllstoffe eignen sich besonders zur Herstellung von Verbundmaterialien.

Bevorzugte anorganische Füllstoffe sind amorphe, kugelförmige, nanopartikuläre Füllstoffe auf der Basis von Oxiden, wie pyrogene Kieselsäure oder Fällungskieselsäure, ZrO₂ und TiO₂ oder Mischoxide aus SiO₂, ZrO₂ und/oder TiO₂, mikrofeine Füllstoffe, wie Quarz-, Glaskeramik-, oder Glaspulver, und röntgenopake Füllstoffe, wie Ytterbiumtrifluorid, nanopartikuläres Tantal(V)-oxid oder Bariumsulfat. Das Ytterbiumtrifluorid hat vorzugsweise eine Partikelgröße von 200 bis 800 nm.

Partikuläre Füllstoffe weisen vorzugsweise eine Partikelgröße von 0,01 bis 15 µm auf. Nanopartikuläre Füllstoffe haben vorzugsweise eine Partikelgröße von 10 bis 100 nm und mikrofeine Füllstoffe vorzugsweise eine Partikelgröße von 0,2 bis 5 µm. Röntgenopake Füllstoffe haben, soweit es sich nicht um nanopartikuläre Füllstoffe handelt, vorzugsweise eine Partikelgröße von 0,2 bis 5 µm.

Wenn nicht anders angegeben, handelt es sich bei allen Partikelgrößen um gewichtsmittlere Partikelgrößen (D50-Werte), wobei die Partikelgrößenbestimmung im Bereich von 0,1 µm bis 1000 µm vorzugsweise mittels statischer Lichtstreuung erfolgt, beispielsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Partikelgrößen kleiner als 0,1 µm können auch anhand von REM- oder TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 Å dickem Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Zur Verbesserung des Verbundes zwischen den Füllstoffpartikeln und der vernetzten Polymerisationsmatrix sind die Füllstoffe vorzugsweise oberflächenmodifiziert. SiO₂-basierende Füllstoffe sind vorzugsweise mit Methacrylat-funktionalisierten Silanen oberflächenmodifiziert, besonders bevorzugt mit 3-Methacryloyloxypropyltrimethoxysilan. Zur Oberflächenmodifizierung von nichtsilikatischen Füllstoffen, z.B. von ZrO₂ oder TiO₂ können auch funktionalisierte saure Phosphate, wie z.B. 10-Methacryloyloxydecyldihydrogenphosphat eingesetzt werden.

Außerdem können die erfindungsgemäßen Zusammensetzungen im Bedarfsfalle weitere Additive sowie Lösungsmittel enthalten. Die Additive sind vorzugsweise aus Stabilisatoren, Kettenübertragungsreagenzien, UV-Absorbern, Farbstoffen oder Pigmenten und Gleitmitteln ausgewählt. Bevorzugte Lösungsmittel sind Wasser, Ethanol, Aceton, Ethylacetat und Mischungen davon.

Die Initiatoren gemäß den Formel (I) zeichnen sich durch eine hohe Photopolymerisationsreaktivität aus, d.h., dass die Bestrahlung schon kleiner Mengen der Verbindungen der Formel (I) zur Auslösung der radikalischen Polymerisation und damit zur Aushärtung der Zusammensetzungen ausreichend sind. Sie können daher in geringen Konzentrationen eingesetzt werden. Die erfindungsgemäßen Zusammensetzungen enthalten, bezogen auf die Gesamtmasse der Zusammensetzung, vorzugsweise 0,001 bis 3 Gew.-%, besonders bevorzugt 0,001 bis 1 Gew.-% und ganz besonders bevorzugt 0,005 bis 0,5 Gew.-% mindestens einer Verbindung der Formel (I).

Die erfindungsgemäßen Verbindungen der allgemeinen Formel (I) eignen sich besonders als Photoinitiatoren zur Herstellung von Polymerisaten, Kompositen, Zementen, Beschichtungsmaterialien, Primern oder Adhäsiven. Sie sind besonders geeignet für Anwendungen im medizinischen Bereich, vor allem zur Herstellung von Dentalwerkstoffen, wie Füllungskompositen, Befestigungszementen, Adhäsiven, Prothesenmaterialien, Verblendmaterialien, Werkstoffen zur Herstellung von Kronen, Brücken, Inlays, Onlays oder von Beschichtungen.

Weitere medizinische Anwendungsgebiete für die erfindungsgemäßen Zusammensetzungen finden sich auf dem Gebiet der Chirurgie, z.B. als Materialien für die Geweberegeneration oder als Werkstoffe zur Herstellung von Gehörhilfen, und in der Augenheilkunde, z.B. zur Herstellung von Intraokularlinsen oder Kontaktlinsen. Unter Materialien für die Geweberegeneration, z.B. von Knochen, werden Polymernetzwerke verstanden, die das Grundgerüst für den Einbau z.B. des Knochenmaterials, beispielsweise von Hydroxylapatit, bilden. Solche Polymernetzwerke lassen sich vorteilhaft unter Verwendung der erfindungsgemäßen Verbindungen der Formel (I) als Photoinitiatoren herstellen. Aufgrund ihrer hohen Aktivität können diese Verbindungen in sehr geringen Konzentrationen eingesetzt werden, was im Hinblick auf die biologische Verträglichkeit der Materialien vorteilhaft ist.

Die Anwendungsmöglichkeiten der erfindungsgemäßen Verbindungen der Formel (I) und der erfindungsgemäßen Zusammensetzungen sind nicht auf den medizinischen Bereich beschränkt. Bei technischen Anwendungen lassen die Verbindungen gemäß der allgemeinen Formel (I) als Photoinitiatoren in der Stereolithographie oder im 3D-Druck, beispielsweise bei der Herstellung von Formkörpern, Prototypen oder Grünkörpern, zur Herstellung von Beschichtungsmaterialien oder in der Mikroelektronik z.B. in der Photoresist-Technologie einsetzen.

Die erfindungsgemäßen Zusammensetzungen enthalten vorzugsweise die folgenden Bestandteile:
(a) 0,001 bis 3 Gew.-%, vorzugsweise 0,001 bis 1,0 Gew.-% und besonders bevorzugt 0,005 bis 0,5 Gew.-% mindestens einer Verbindung der allgemeinen Formel (I),
(b) 1 bis 99,9 Gew.-%, vorzugsweise 5 bis 95 Gew.-% und besonders bevorzugt 10 bis 90 Gew.-% mindestens eines radikalisch polymerisierbaren Monomers,
(c) 0 bis 85 Gew.-%, vorzugsweise 5 bis 80 Gew.-% und besonders bevorzugt 10 bis 75 Gew.-% mindestens eines Füllstoffs und
(d) 0 bis 70 Gew.-%, vorzugsweise 0,1 bis 60 Gew.-% und besonders bevorzugt 0,1 bis 50 Gew.-% eines oder mehrerer Additive.

Alle Prozentangaben hierin beziehen sich, wenn nicht anders angegeben, auf die Gesamtmasse der Zusammensetzung.

Zusammensetzungen zur Verwendung als Zemente und insbesondere als dentale Zemente enthalten bevorzugt:
(a) 0,001 bis 3 Gew.-%, vorzugsweise 0,001 bis 1,0 Gew.-% und besonders bevorzugt 0,005 bis 0,5 Gew.-% mindestens einer Verbindung der allgemeinen Formel (I),
(b) 5 bis 70 Gew.-%, vorzugsweise 10 bis 60 Gew.-% und besonders bevorzugt 20 bis 55 Gew.-% mindestens eines radikalisch polymerisierbaren Monomers,
(c) 20 bis 80 Gew.-%, vorzugsweise 20 bis 70 Gew.-% und besonders bevorzugt 40 bis 60 Gew.-% mindestens eines Füllstoffs und
(d) 0,1 bis 10 Gew.-%, vorzugsweise 1,0 bis 10 Gew.-% und besonders bevorzugt 1,00 bis 5 Gew.-% eines oder mehrerer Additive.

Zusammensetzungen zur Verwendung als Komposite und insbesondere als dentale Füllungskomposite enthalten bevorzugt:
(a) 0,001 bis 3 Gew.-%, vorzugsweise 0,001 bis 1,0 Gew.-% und besonders bevorzugt 0,005 bis 0,5 Gew.-% mindestens einer Verbindung der allgemeinen Formel (I),
(b) 5 bis 70 Gew.-%, vorzugsweise 10 bis 50 Gew.-% und besonders bevorzugt 20 bis 40 Gew.-% mindestens eines radikalisch polymerisierbaren Monomers,
(c) 30 bis 85 Gew.-%, vorzugsweise 40 bis 80 Gew.-% und besonders bevorzugt 45 bis 77 Gew.-% mindestens eines Füllstoffs und
(d) 0,1 bis 10 Gew.-%, vorzugsweise 0,5 bis 5 Gew.-% und besonders bevorzugt 0,5 bis 3 Gew.-% eines oder mehrerer Additive.

Zusammensetzungen zur Verwendung als Beschichtungsmaterialien enthalten bevorzugt:
(a) 0,001 bis 5 Gew.-%, vorzugsweise 0,001 bis 3,0 Gew.-% und besonders bevorzugt 0,05 bis 3,0 Gew.-% mindestens einer Verbindung der allgemeinen Formel (I),
(b) 10 bis 99,9 Gew.-%, vorzugsweise 15 bis 99,9 Gew.-% und besonders bevorzugt 30 bis 99,9 Gew.-% mindestens eines radikalisch polymerisierbaren Monomers,
(c) 0 bis 70 Gew.-%, vorzugsweise 0 bis 60 Gew.-% und besonders bevorzugt 0 bis 20 Gew.-% mindestens eines nanopartikulären Füllstoffs und
(d) 0,1 bis 10 Gew.-%, vorzugsweise 0,1 bis 5 Gew.-% und besonders bevorzugt 0,1 bis 3 Gew.-% eines oder mehrerer Additive.
(e) 0 bis 70 Gew.-%, vorzugsweise 0 bis 60 Gew.-% und besonders bevorzugt 0 bis 50 Gew.-% Lösungsmittel.

Zusammensetzungen zur Verwendung als Adhäsive und insbesondere als dentale Adhäsive enthalten bevorzugt:
(a) 0,001 bis 5 Gew.-%, vorzugsweise 0,001 bis 3,0 Gew.-% und besonders bevorzugt 0,05 bis 1,0 Gew.-% mindestens einer Verbindung der allgemeinen Formel (I),
(b1) 1 bis 95 Gew.-%, vorzugsweise 5 bis 80 Gew.-% und besonders bevorzugt 20 bis 80 Gew.-% mindestens eines radikalisch polymerisierbaren Monomers,
(b2) 1 bis 20 Gew.-% vorzugsweise 1,0 bis 15 Gew.-% und besonders bevorzugt 2 bis 15 Gew.-% mindestens eines radikalisch polymerisierbaren Haftmonomers,
(c) 0 bis 40 Gew.-%, vorzugsweise 0 bis 30 Gew.-% und besonders bevorzugt 1 bis 10 Gew.-% mindestens eines nanopartikulären Füllstoffs und
(d) 0,1 bis 10 Gew.-%, vorzugsweise 0,1 bis 5 Gew.-% und besonders bevorzugt 0,3 bis 5 Gew.-% eines oder mehrerer Additive.
(e) 0 bis 70 Gew.-%, vorzugsweise 5 bis 60 Gew.-% und besonders bevorzugt 10 bis 55 Gew.-% Lösungsmittel.

Zusammensetzungen zur Verwendung als Werkstoffe und insbesondere als dentale Werkstoffe für die Stereolithographie oder den 3D-Druck enthalten bevorzugt:
(a) 0,001 bis 3 Gew.-%, vorzugsweise 0,001 bis 1,0 Gew.-% und besonders bevorzugt 0,005 bis 1,0 Gew.-% mindestens einer Verbindung der allgemeinen Formel (I),
(b) 1 bis 99,9 Gew.-%, vorzugsweise 20 bis 98 Gew.-% und besonders bevorzugt 30 bis 95 Gew.-% mindestens eines radikalisch polymerisierbaren Monomers oder Harzes,
(c) 0 bis 85 Gew.-%, vorzugsweise 1 bis 70 Gew.-% und besonders bevorzugt 3 bis 60 Gew.-% mindestens eines Füllstoffs und
(d) 0,1 bis 70 Gew.-%, vorzugsweise 0,5 bis 60 Gew.-% und besonders bevorzugt 1,0 bis 50 Gew.-% eines oder mehrerer Additive.

Erfindungsgemäße Zusammensetzungen für dentale Zwecke eignen sich besonders zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch nicht-therapeutisch (extraoral) eingesetzt werden, beispielsweise bei der Herstellung oder Reparatur von Dentalrestaurationen, wie Prothesen, künstlichen Zähnen, Inlays, Onlays, Kronen und Brücken.

Gegenstand der Offenbarung ist auch die Verwendung einer Verbindung gemäß Formel (I) zur Herstellung eines radikalisch polymerisierbaren Werkstoffs, vorzugsweise eines medizintechnischen und insbesondere eines zahnmedizinischen Werkstoffs.

Die Erfindung wird im Folgenden anhand von Beispielen näher erläutert.

### Beispiel 1

### Synthese des 1,2-Bis(trismesitoylgermyl)terephtalat 1

### 1. Stufe: Synthese des Tris(mesitoyl)germenolat TMGe (Methode A)

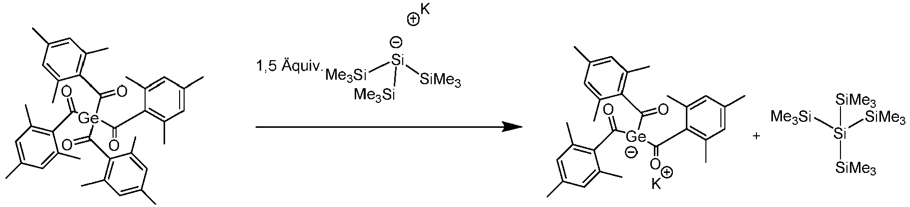

In einen Kolben, der 2,18 g (6,79 mmol; 1,5 Äquiv.) Tetrakis(trimethylsilyl)silan (Me₃Si)₄Si und 0,76 g Kalium-tert.butylat KO*t*Bu (6,79 mmol; 1,5 Äquiv.) enthielt, wurden 25 mL von Dimethoxyethan (DME) zugegeben. Die Reaktionsmischung zur Bildung des Kaliumsilanides wurde dann für eine 1 h gerührt. In einem 2. Kolben wurden 3,00 g (4,53 mmol; 1,0 Äquiv.) Tetrakis(mesitoyl)german in 30 mL DME gelöst. Dazu wurde die Kaliumsilanidlösung mittels einer Spritze langsam zugegeben und die Reaktionslösung für 2 h gerührt. Die Reaktion wurde mittels NMR-Spektroskopie verfolgt. Die so erhaltene Tris(mesitoyl)germenolat-Lösung kann für weitere Umsetzungen bei -30 °C über Wochen gelagert werden.

### 1. Stufe: Synthese des Tris(mesitoyl)germenolat TMGe (Methode B)

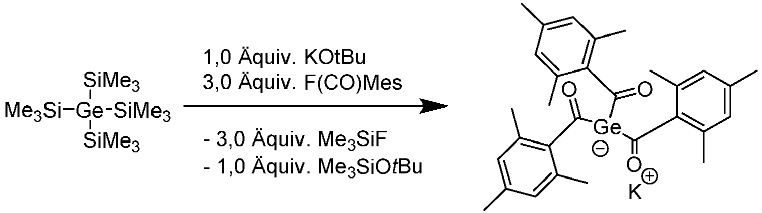

In einen Kolben, der 3,00 g (8,21 mmol) Tetrakis(trimethylsilyl)german (Me₃Si)₄Ge und 1,01 g KO*t*Bu (9,03 mmol; 1,1 Äquiv.) enthielt, wurden 35 mL DME zugegeben. Die Reaktionsmischung wurde dann für eine 1 h gerührt. Danach gab man 1.36 g (8.21 mmol; 1,0 Äquiv.) Mesitoylfluorid zu und rührt weitere 10 Minuten. Man wiederholte die Zugabe 2 mal mit der gleichen Menge an Mesitoylfluorid (total 2,73 g, 2,0 Äquiv.) und rührte dann noch für 2 h. Die so erhaltene Tris(mesitoyl)germenolat-Lösung kann für weitere Umsetzungen bei -30 °C über Wochen gelagert werden. Ausbeute:
Methode A: 2,8 g (96 %). (das Germenolat TMGe enthält ein Molekül DME) Methode B: 2,9 g (99 %)
¹H-NMR: δ_{H} (400 MHz, THF-D₈): 6,39 (s, 6H, Aryl-H), 3,43 (s, 3,2H, CH₂), 3,27 (s, 4,5H, CH₃), 2,15 (s, 9H, pCH₃), 2,04 (s, 18H, oCH₃).
¹³C-NMR: δ_{C} (100 MHz, THF-D₈): 262,77 (GeCOMes), 148,62 (Aryl-C1), 135,18 (Aryl-C2), 131,63 (Aryl-C3) 128,44 (Aryl-*C*4), 72,77 (-CH₂-), 58,95(-CH₃), 21,30 (Aryl-pCH₃), 20,03 (Aryl-*o***C**H₃).
Schmelzpunkt: 154-156 °C.
UV-VIS: λ [nm] (ε[Lmol⁻¹ cm⁻¹]) = 427 (3454), 353 (3030).
IR: v [cm⁻¹] = 1604, 1590, 1555, 1535 (m, vC=O).
Elementaranalyse: Berechnet: C: 63,47 %, H: 6,74 %; Gefunden: C: 63,53 %, 6,75%.

### 2. Stufe: Synthese des 1,2-Bis(trismesitoylgermyl)terephtalat 1

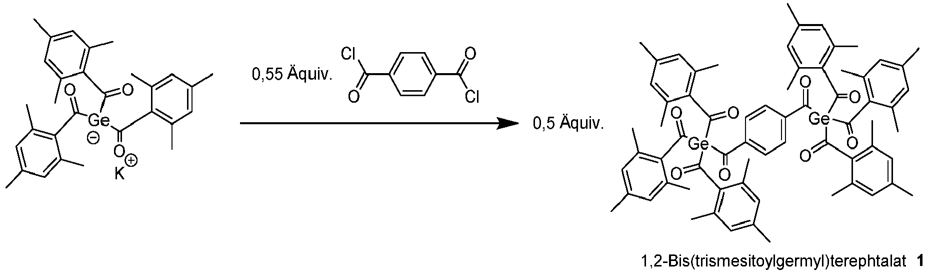

Zu der nach der Methode A hergestellten Lösung des Tris(mesitoyl)germenolates wurden unter Rühren 0,51 g (2,55 mmol, 0,66 Äquiv.) Terephthaloylchlorid gelöst in 60 mL Toluol bei -30 °C zugegeben. Die Reaktionslösung wurde langsam auf Raumtemperatur erwärmt, wobei die NMR-spektroskopische Reaktionsverfolgung die Bildung des 1,2-Bis(trismesitoylgermyl)terephtalat **1** zeigte. Nach wässriger Aufarbeitung des Reaktionsansatzes mit 50 mL gesättigter NH₄Cl-Lösung wurde die organische Phase abgetrennt und über wasserfreiem Na₂SO₄ getrocknet. Danach wurde die Lösung abfiltriert und die flüchtigen Komponenten im Vakuum abgezogen. Der feste Rückstand wurde aus Aceton umkristallisiert und man erhielt 1,37g (52 % Ausbeute) des 1,2-Bis(trismesitoylgermyl)terephtalat **1** als gelben kristallinen Feststoff.

¹H-NMR: δ_{H} (400 MHz, CDCl₃): 7,22 (s, 4H, Aryl-H), 6,56 (s, 12H, Aryl-H), 2,15 (s, 18H, pCH₃), 2,11 (s, 36H, oCH₃).

¹³C-NMR: δ_{C} (100 MHz, CDCl₃): 231,61, 222,24 (GeC=O), 141,94, 141,33, 140,12, 133,11, 128,87, 128,61 (Aryl-C), 21,23 (Aryl-*p***C**H₃), 19,35 (Aryl-*o***C**H₃). Schmelzpunkt: 245-247°C.

UV-VIS: λ [nm] (ε[L mol⁻¹ cm⁻¹]) = 434sh (1645), 377 (5252).

IR: v [cm⁻¹] = 1658, 1643, 1632, 1619, 1607 (m, vC=O).

Elementaranalyse: Berechnet: C: 70,38 %, H: 6,08 %; Gefunden: C: 70,42 %, 6,10%.

Das hergestellte 1,2-Bis(trismesitoylgermyl)terephtalat **1** zeigte mit dem Extinktionskoeffizienten von 5252 L mol⁻¹ cm⁻¹ der Absorptionsbande bei 377 nm einen deutlich höheren Wert im Vergleich zu dem aus dem Stand der Technik bekannten Tetrakis(mesitoyl)german mit nur ε = 1984 L mol⁻¹ cm⁻¹ der Bande bei 376 nm. Der Extinktionskoeffizient war auch deutlich größer als der des hoch wirksamen, kommerziellen Photoinitiators Bis(4-methoxybenzoyl)diethylgermanium mit ε = 724 L mol⁻¹ cm⁻¹ der Bande bei 408 nm.

### Beispiel 2

### Synthese des 1,2-Bis(trismesitoylstannvy)tereophtalat 2

### 1. Stufe: Synthese des Tris(mesitoyl)stannenolat TMSn (Methode A)

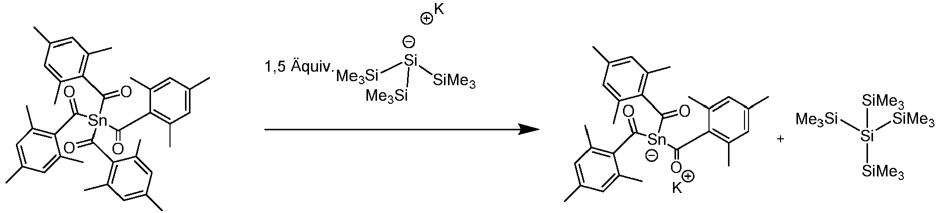

In einen Kolben, der 2,04 g (6.36 mmol; 1,5 Äquiv.) Tetrakis(trimethylsilyl)silan (Me₃Si)₄Si und 0,71 g Kalium-tert.butylat KO*t*Bu (6,36 mmol; 1,5 Äquiv.) enthielt, wurden 25 mL of Dimethoxyethan (DME) zugegeben. Die Reaktionsmischung zur Bildung des Kaliumsilanides wurde dann für eine 1 h gerührt. In einem 2. Kolben wurden 3,00 g (4,24 mmol; 1,0 Äquiv.) Tetrakis(mesitoyl)stannan in 30 mL DME gelöst. Dazu wurde die Kaliumsilanidlösung mittels einer Spritze langsam zugegeben und die Reaktionslösung für 2 h gerührt. Die Reaktion wurde mittels NMR-Spektroskopie verfolgt. Die so erhaltene Tris(mesitoyl)stannenolat-Lösung konnte für weitere Umsetzungen bei -30 °C über Wochen gelagert werden. Um Kristalle zu erhalten, wurde 1 Äquiv. des Kronenethers 18-Krone-6 zugesetzt.

### 1. Stufe: Synthese des Tris(mesitoyl)stannenolat TMSn (Methode B)

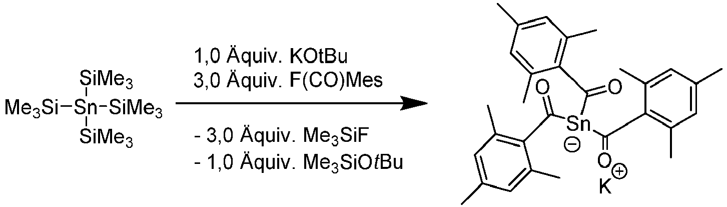

In einen Kolben, der 3,00 g (7.29 mmol) Tetrakis(trimethylsilyl)stannan (Me₃Si)₄Sn und 0,90 g KO*t*Bu (8,02 mmol; 1,1 Äquiv.) enthält, wurden 35 mL DME zugegeben. Die Reaktionsmischung wurde dann für eine 1 h gerührt. Danach gab man 3,64 g (21,87 mmol; 3,0 Äquiv.) Mesitoylfluorid zu und rührte für weitere 30 Minuten. Die so erhaltene Tris(mesitoyl)stannenolat-Lösung konnte für weitere Umsetzungen bei -30 °C über Wochen gelagert werden. Um Kristalle zu erhalten, wurde 1 Äquiv. des Kronenethers 18-Krone-6 zugesetzt.
Ausbeute:
Methode A: 3,1 g (85 %) (das Stannenolat TMSn enthält ein Molekül 18-Krone-6)
Methode B: 5,3 g (84 %)
¹H-NMR: δ_{H} (400 MHz, C₆D₆): 6,68 (s, 6H, Aryl-H), 3,28 (s, 24H (CH₂-CH₂-O)-), 2,42 (s, 18H, *o*CH₃), 2,17 (s, 9H, *p*CH₃).
¹³C-NMR: δ_{C} (100 MHz, C₆D₆): 286,90 (GeCOMes), 151,17 (Aryl-C1), 135,41 (Aryl-C2), 130,76 (Aryl-C3) 128,91 (Aryl-C4), 70,23 (CH₂-CH₂-O)-, 21,19 (Aryl-*p***C**H₃), 19,76 (Aryl-*o***C**H₃).
¹¹⁹Sn (C₆D₆): δ [ppm] = 450,04 (SnCOMes).
Schmelzpunkt: 165-167 °C.
UV-VIS: λ [nm] (ε[L mol⁻¹ cm⁻¹]) = 427 (3454), 353 (3030).
IR: v [cm⁻¹] = 1607, 1581, 1562 (m, vC=O).
Elementaranalyse: Berechnet: C: 58,41 %, H: 6,65 %; Gefunden: C: 58,43 %, 6,69%.

### 2. Stufe: Synthese des 1,2-Bis(trismesitoylstannyl)terephtalat 2

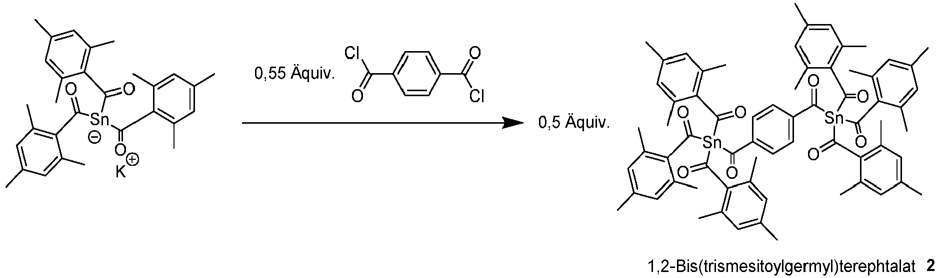

Zu der nach der Methode A hergestellten Lösung des Tris(mesitoyl)stannenolates wurden unter Rühren 0,47 g (2.33 mmol, 0,55 Äquiv.) Terephthaloylchlorid gelöst in 60 mL Toluol bei -30 °C zugegeben. Die Reaktionslösung wurde langsam auf Raumtemperatur erwärmt, wobei die NMR-spektroskopische Reaktionsverfolgung die Bildung des Octaacylstannans 2 zeigte. Danach wurden von Reaktionsmischung die flüchtigen Komponenten im Vakuum abgezogen. Der feste Rückstand wurde in Toluol gelöst, die unlöslichen Salzanteile abfiltriert und das Filtrat im Vakuum zur Trockene eingeengt. Schließlich wurde der feste Rückstand in einer Mischung aus Dichlormethan und Diethylether (2:1) umkristallisiert und man erhielt 1,60 g (60 % Ausbeute) des 1,2-Bis(trismesitoylstannyl)terephtalats **2** als roten kristallinen Feststoff.

¹H-NMR: δ_{H} (400 MHz, CDCl₃): 7,48 (s, 4H, Aryl-H), 6,61 (s, 12H, Aryl-H), 2,18 (s, 18H, pCH₃), 2,10 (s, 36H, oCH₃).

¹³C-NMR: 241,98, 235.36 (Sn*C*=O), 143,53, 143,29, 139,85, 131,69, 128,98, 128,87 (Aryl-C), 21.12(Aryl-*p***C**H₃), 18,75 (Aryl-*o***C**H₃).

Schmelzpunkt: 175-177°C.

UV-VIS: λ [nm] (ε[L mol⁻¹ cm⁻¹]) = 470sh (1000), 400 (3200).

IR: v [cm⁻¹] = 1665, 1625, 1603, (m, vC=O).

Elementaranalyse: Berechnet: C: 65,20 %, H: 5,63 %; Gefunden: C: 65,23 %, 5,66%.

Das hergestellte 1,2.Bis(trismesitoylstannyl)terephtalat **2** zeigte mit dem Extinktionskoeffizienten von 4000 L mol⁻¹ cm⁻¹ der Absorptionsbande bei 400 nm einen deutlich höheren Wert im Vergleich zu dem aus dem Stand der Technik bekannten Tetrakis(mesitoyl)stannan mit ε = 1736 L mol⁻¹ cm⁻¹ der Bande bei 398 nm oder dem hoch wirksamen, kommerziellen Photoinitiator Bis(4-methoxybenzoyl)diethylgermanium mit ε = 724 L mol⁻¹ cm⁻¹ der Bande bei 408 nm.

### Beispiel 3

### Synthese des 1,3,5-Tris(trismesitoylgermyl)tricarbonylbenzol 3

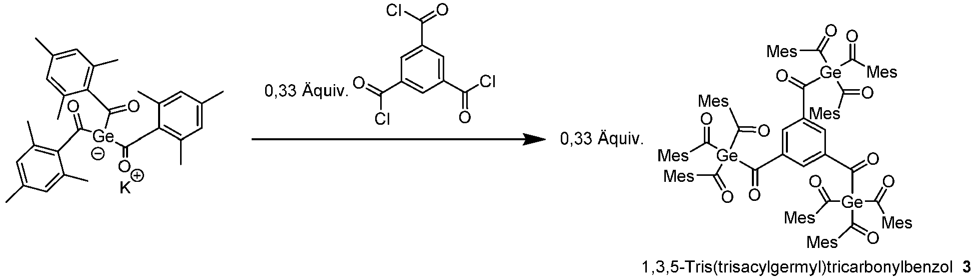

### Methode A:

Das Tris(mesitoyl)germenolat wurde analog Beispiel 1 nach Methode A mit 2,18 g (6,79 mmol; 1,5 C) (Me₃Si)₄Si, 0,76 g KO*t*Bu (6,79 mmol; 1,5 Äquiv.), 3,00 g (4,53 mmol; 1,0 Äquiv.) Tetrakis(mesitoyl)german und 25 mL von Dimethoxyethan (DME) hergestellt. Danach wurde diese Lösung zu einer Mischung aus 0,40 g (1,50 mmol, 0,33 Äquiv.) von Benzol-1,3,5-tricarbonyltrichlorid und 60 mL Toluol bei -30°C über eine Spritze zugegeben. Nach vollständiger Zugabe wurde langsam auf Raumtemperatur erwärmt und die Reaktion mittels NMR-Spektroskopie verfolgt, was die Bildung von 3 anzeigte. Nach wässriger Aufarbeitung des Reaktionsansatzes mit 50 mL gesättigter NH₄Cl-Lösung wurde die organische Phase abgetrennt und über wasserfreiem Na₂SO₄ getrocknet. Danach wurde die Lösung abfiltriert und die flüchtigen Komponenten im Vakuum abgezogen. Der feste Rückstand wurde aus Aceton umkristallisiert und man erhielt 1,20 g (52 % Ausbeute) des 1,3,5-Tris(trismesitoylgermyl)-tricarbonylbenzol 3 als gelben kristallinen analysenreinen Feststoff.

### Methode B:

Das Tris(mesitoyl)germenolat wurde analog Beispiel 1 nach Methode B mit 3,00 g (8,21 mmol) (Me₃Si)₄Ge, 1,01g KO*t*Bu (9,03 mmol; 1,1 Äquiv.), 4,09 g (24,63 mmol; 3,0 Äquiv.) Mesitoylfluorid und 35 mL DME hergestellt. Danach wurde diese Lösung zu einer Mischung aus 0,72 g (2,71 mmol, 0,33 Äquiv.) Benzol-1,3,5-tricarbonyl trichlorid und 60 mL Toluol bei -30°C über eine Spritze zugegeben. Nach vollständiger Zugabe wurde langsam auf Raumtemperatur erwärmt und die Reaktion mittels NMR-Spektroskopie verfolgt, was die Bildung von 3 anzeigte. Nach wässriger Aufarbeitung des Reaktionsansatzes mit 50 mL gesättigter NH₄Cl-Lösung wurde die organische Phase abgetrennt und über wasserfreiem Na₂SO₄ getrocknet. Danach wurde die Lösung abfiltriert und die flüchtigen Komponenten im Vakuum abgezogen. Der feste Rückstand wurde aus Aceton umkristallisiert und man erhielt 2,86 g (62 % Ausbeute) des Tris(trismesitoylgermyl)tricarbonylbenzol 3 als gelben kristallinen analysenreinen Feststoff.

¹H-NMR: δ_{H} (400 MHz, CDCl₃): 6,05 (s, 3H, Aryl-H), 6,35 (s, 18H, Aryl-H), 2,14 (s, 27H, pCH₃), 2,12 (s, 54H, oCH₃).

¹³C-NMR: 231,05, 219.52 (GeC=O), 141,47, 140,67, 139,78, 133,19, 132,66, 128,94 (Aryl-C), 21,26 (Aryl-*p***C**H₃), 19,41 (Aryl-*o***C**H₃).

Schmelzpunkt: 189-192°C.

UV-VIS: λ [nm] (ε[L mol⁻¹ cm⁻¹]) = 434sh (1776), 381 (5142).

IR: v [cm⁻¹] = 1654, 1639, 1606 (m, vC=O)

Elementaranalyse: Berechnet: C: 69,87 %, H: 6,04 %; Gefunden: C: 69,89 %, 6,05%.

Das hergestellte Tris(trismesitoylgermyl)tricarbonylbenzol 3 zeigte mit dem Extinktionskoeffizienten von 5142 L mol⁻¹ cm⁻¹ der Absorptionsbande bei 381 nm einen deutlich höheren Wert im Vergleich zu dem aus dem Stand der Technik bekannten Tetrakis(mesitoyl)german mit nur ε = 1984 L mol⁻¹ cm⁻¹ der Bande bei 376 nm oder dem hoch wirksamen, kommerziellen Photoinitiator Bis(4-methoxybenzoyl)diethylgermanium mit ε = 724 L mol⁻¹ cm⁻¹ der Bande bei 408 nm.

### Beispiel 4

### Synthese des 1,4-Bis(trismesitoylgermyl)butan 4

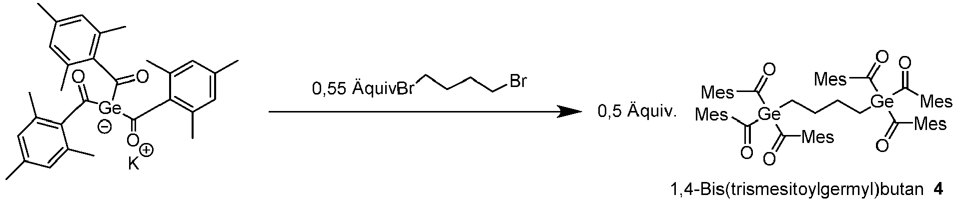

### Methode A:

Das Tris(mesitoyl)germenolat wurde analog Beispiel 1 nach Methode A mit 2,18 g (6,79 mmol; 1,5 C) (Me₃Si)₄Si, 0,76 g KO*t*Bu (6,79 mmol; 1,5 Äquiv.), 3,00 g (4,53 mmol; 1,0 Äquiv.) Tetrakis(mesitoyl)german und 25 mL von Dimethoxyethan (DME) hergestellt. Danach wurde diese Lösung zu einer Mischung aus 0,54 g (2,49 mmol, 0,55 Äquiv.) von 1,4-Dibrombutan und 60 mL Toluol bei -30°C über eine Spritze zugegeben. Nach vollständiger Zugabe wurde langsam auf Raumtemperatur erwärmt und die Reaktion mittels NMR-Spektroskopie verfolgt, was die Bildung von 4 anzeigte. Nach wässriger Aufarbeitung des Reaktionsansatzes mit 50 mL gesättigter NH₄Cl-Lösung wurde die organische Phase abgetrennt und über wasserfreiem Na₂SO₄ getrocknet. Danach wurde die Lösung abfiltriert und die flüchtigen Komponenten im Vakuum abgezogen. Der feste Rückstand wurde aus n-Pentan umkristallisiert und man erhielt 1,60 g (65 % Ausbeute) des 1,4-Bis(trismesitoylgermyl)butan 4 als gelben kristallinen analysenreinen Feststoff.

### Methode B:

Das Tris(mesitoyl)germenolat wurde analog Beispiel 1 nach Methode B mit 3,00 g (8,21 mmol) (Me₃Si)₄Ge, 1,01g KO*t*Bu (9,03 mmol; 1,1 Äquiv.), 4,09 g (24,63 mmol; 3,0 Äquiv.) Mesitoylfluorid und 35 mL DME hergestellt. Danach wurde diese Lösung zu einer Mischung aus 0,98 g (4,51 mmol, 0,55 Äquiv.) von 1,4-Dibrombutan und 60 mL Toluol bei -30°C über eine Spritze zugegeben. Nach vollständiger Zugabe wurde langsam auf Raumtemperatur erwärmt und die Reaktion mittels NMR-Spektroskopie verfolgt, was die Bildung von 3 anzeigte. Nach wässriger Aufarbeitung des Reaktionsansatzes mit 50 mL gesättigter NH₄Cl-Lösung wurde die organische Phase abgetrennt und über wasserfreiem Na₂SO₄ getrocknet. Danach wurde die Lösung abfiltriert und die flüchtigen Komponenten im Vakuum abgezogen. Der feste Rückstand wurde aus Aceton umkristallisiert und man erhielt 3,34 g (75 % Ausbeute) des 1,4-Bis(trismesitoylgermyl)butan 4 als gelben kristallinen analysenreinen Feststoff.

¹H-NMR: δ_{H} (400 MHz, CDCl₃): 6,68 (s, 12H, Aryl-H), 2,26 (s, 18H, pCH₃), 2,04 (s, 36H, *o*CH₃), (bs, 8H, -(CH₂)₄-).

¹³C-NMR: 237,45 (GeC=O), 142,16, 139,30, 132,49, 128,83, (Aryl-C), 27,52, 16,75 (CH₂-C) 21,26 (Aryl-*p***C**H₃), 19,10 (Aryl-*oC*H₃).

Schmelzpunkt: 180-181 °C.

UV-VIS: λ [nm] (ε[L mol⁻¹ cm⁻¹]) = 400 (1982), 382 (2628).

IR: v [cm⁻¹] = 1650, 1632, 1627, 1606 (m, vC=O)

Elementaranalyse: Berechnet: C: 70,88 %, H: 6,88 %; Gefunden: C: 70,89 %, 6,87%.

Das hergestellte 1,4-Bis(trismesitoylgermyl)butan **4** zeigte mit dem Extinktionskoeffizienten von 2628 L mol⁻¹ cm⁻¹ der Absorptionsbande bei 382 nm einen deutlich höheren Wert im Vergleich zu dem nach dem Stand der Technik bekannten Tetrakis(mesitoyl)german mit nur ε = 1984 L mol⁻¹ cm⁻¹ der Bande bei 376 nm oder dem hoch wirksamen, kommerziellen Photoinitiator Bis(4-methoxybenzoyl)diethylgermanium mit ε = 724 L mol⁻¹ cm⁻¹ der Bande bei 408 nm.

### Beispiel 5

### Herstellung von lichthärtbaren Kompositen unter Verwendung von 1,4-Bis(trismesitoylgermyl)butan 4 aus Beispiel 4

Aus einer Mischung (Angaben in Masse-%) der Dimethacrylate Bis-GMA (Additionsprodukt aus Methacrylsäure und Bisphenol-A-diglycidylether) und Bis(methacry-loyloxymethyl)tricyclo-[5.2.1.0^{2,6}]decan (DCP), dem jeweiligen Photoinitiator und Füllstoff (silanisierter Glasfüller GM 27884, 0,7 µm, Schott), wurden die lichthärtenden Komposite **C1** und **C2** (Referenzkomposit) mittels eines Walzenstuhles (Modell "Exakt", Exakt Apparatebau, Norderstedt) hergestellt (Tabelle 1).

**Tabelle 1: Zusammensetzung der Komposite C1 und C2**

| **Komponente** | **C1** | **C2^{*)}** |
|---|---|---|
| Photoinitiator: 1,4-Bis(trismesitoylgermyl)butan **4** | 0,05 | - |
| Photoinitiator: Bis(4-methoxybenzoyl)¬diethyl¬germanium | - | 0,05 |
| DCP | 17,47 | 17,47 |
| Bis-GMA | 17,48 | 17,48 |
| Glasfüller GM 27884, sil. | 65,00 | 65,00 |

| | | |
|---|---|---|
| *) Vergleichsbeispiel | | |

Die Bestimmung der Biegefestigkeit (BF) und des Biege-E-Moduls (BM) der Materialien erfolgte nach der ISO-Norm ISO-4049 (Dentistry - Polymer-based filling, restorative and luting materials). Hierzu wurden Prüfkörper präpariert, die 2 mal 40 s in einer Bestrahlungskammer (Fa. Hönle, Gräfelfing) gleichzeitig mit Licht einer Wellenlänge von 410 nm und von 460 nm bestrahlt und damit ausgehärtet wurden. Biegefestigkeit (BF) und Biege-E-Modul (BM) wurden nach 24 h Lagerung in Wasser mit einer Temperatur von 37 °C (WL) gemessen (Tabelle 2).

**Tabelle 2: Biegefestigkeit (BF, MPa) und des Biege-E-Moduls (BM, GPa) der polymerisierten Komposite C1 und C2**

| | **C1** | **C2^{*)}** |
|---|---|---|
| BF, WL | 104,6±13,2 | 122,0±13,1 |
| BM, WL | 5,30±0,39 | 6,72±0,84 |

| | | |
|---|---|---|
| *) Vergleichsbeispiel | | |

Die Ergebnisse in Tabelle 2 belegen die sehr gute polymerisationsauslösende Wirkung des erfindungsgemäßen 1,4-Bis(trismesitoylgermyl)butan **4** auch im Vergleich zu dem kommerziellen Photoinitiator Bis(4-methoxybenzoyl)¬diethyl¬germanium, der deutlich schwerer zugänglich und dementsprechend sehr teuer ist.

## Patentansprüche

1. Verbindung gemäß der allgemeinen Formel (I) 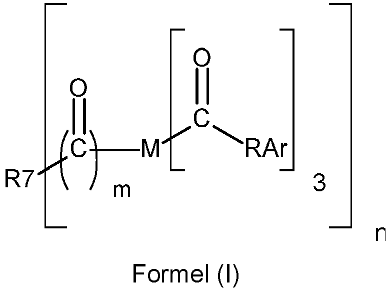 in der die Variablen die folgenden Bedeutungen haben:
M Ge oder Sn
RAr 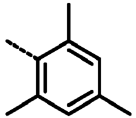
R⁷ 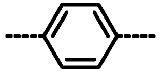
n 2
m 1
oder
M Ge
RAr 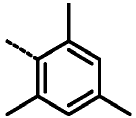
R⁷ 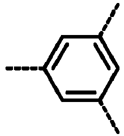
n 3
m 1
oder
M Ge
RAr 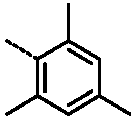
R⁷ C₂-C₈-Alkylen
n 2
m 0.

2. Verbindung nach Anspruch 1, wobei die Variablen der Formel (I) die folgenden Bedeutungen haben:
M Ge
RAr 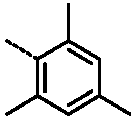
R⁷ -C₄H₈-
n 2
m 0.

3. Zusammensetzung, die mindestens eine Verbindung gemäß einem der Ansprüche 1 bis 2 und mindestens ein polymerisierbares Monomer enthält.

4. Zusammensetzung nach Anspruch 3, die als polymerisierbares Monomer ein radikalisch polymerisierbares Monomer enthält.

5. Zusammensetzung nach Anspruch 3 oder 4, die 0,001 bis 3 Gew.-% einer Verbindung gemäß einem der Ansprüche 1 bis 2 enthält, bezogen auf die Gesamtmasse der Zusammensetzung.

6. Zusammensetzung nach Anspruch 3 oder 4, die 0,001bis 1 Gew.-% einer Verbindung gemäß einem der Ansprüche 1 bis 2 enthält, bezogen auf die Gesamtmasse der Zusammensetzung.

7. Zusammensetzung nach Anspruch 3 oder 4, die 0,005 bis 0,5 Gew.-% einer Verbindung gemäß einem der Ansprüche 1 bis 2 enthält, bezogen auf die Gesamtmasse der Zusammensetzung.

8. Zusammensetzung nach einem der Ansprüche 4 bis 7, die als radikalisch polymerisierbares Monomer mindestens ein mono- oder ein multifunktionelles (Meth)acrylate oder eine Mischung davon enthält.

9. Zusammensetzung nach einem der Ansprüche 3 bis 8, die
(a) 0,001 bis 3 Gew.-% mindestens einer Verbindung der allgemeinen Formel (I),
(b) 1 bis 99,9 Gew.-% mindestens eines radikalisch polymerisierbaren Monomers,
(c) 0 bis 85 Gew.-% mindestens eines Füllstoffs und
(d) 0 bis 70 Gew.-% eines oder mehrerer Additive
enthält, jeweils bezogen auf die Gesamtmasse der Zusammensetzung.

10. Zusammensetzung nach einem der Ansprüche 3 bis 8, die
(a) 0,001 bis 1,0 Gew.-% mindestens einer Verbindung der allgemeinen Formel (I),
(b) 5 bis 95 Gew.-% mindestens eines radikalisch polymerisierbaren Monomers,
(c) 5 bis 80 Gew.-% mindestens eines Füllstoffs und
(d) 0,1 bis 60 Gew.-% eines oder mehrerer Additive
enthält, jeweils bezogen auf die Gesamtmasse der Zusammensetzung.

11. Zusammensetzung nach einem der Ansprüche 3 bis 8, die
(a) 0,005 bis 0,5 Gew.-% mindestens einer Verbindung der allgemeinen Formel (I),
(b) 10 bis 90 Gew.-% mindestens eines radikalisch polymerisierbaren Monomers,
(c) 10 bis 75 Gew.-% mindestens eines Füllstoffs und
(d) 0,1 bis 50 Gew.-% eines oder mehrerer Additive
enthält, jeweils bezogen auf die Gesamtmasse der Zusammensetzung.

12. Zusammensetzung nach einem der Ansprüche 3 bis 11 zur therapeutischen Anwendung als Dentalwerkstoff.

13. Zusammensetzung zur therapeutischen Anwendung nach Anspruch 12 als dentaler Zement, Füllungskomposit oder Verblendmaterial.

14. Nicht therapeutische Verwendung einer Zusammensetzung gemäß einem der Ansprüche 3 bis 11 zur Herstellung oder Reparatur von Dentalrestaurationen, Prothesen, künstlichen Zähnen, Inlays, Onlays, Kronen oder Brücken.

15. Verwendung einer Verbindung gemäß einem der Ansprüche 1 bis 2 als Photoinitiator.

## Claims

1. Compound according to general formula (I) 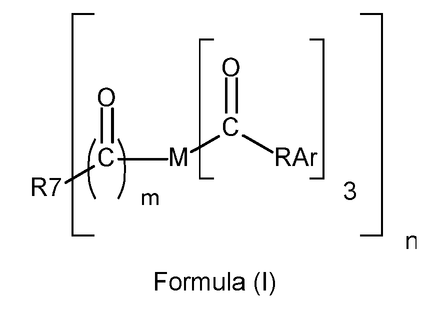 in which the variables have the following meanings:
M Ge or Sn
RAr 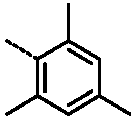
R⁷ 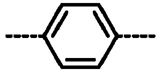
n 2
m 1
or
M Ge
RAr 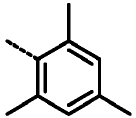
R⁷ 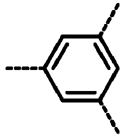
n 3
m 1
or
M Ge
RAr 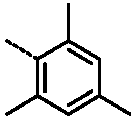
R⁷ C₂-C₈ alkylene
n 2
m 0.

2. Compound according to claim 1, wherein the variables of formula (I) have the following meanings:
M Ge
RAr 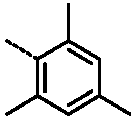
R⁷ -C₄H₈-
n 2
m 0.

3. Composition which comprises at least one compound according to one of claims 1 to 2 and at least one polymerizable monomer.

4. Composition according to claim 3, which comprises a radically polymerizable monomer as polymerizable monomer.

5. Composition according to claim 3 or 4, which comprises 0.001 to 3 wt.-% of a compound according to one of claims 1 to 2, relative to the total mass of the composition.

6. Composition according to claim 3 or 4, which comprises 0.001 to 1 wt.-% of a compound according to one of claims 1 to 2, relative to the total mass of the composition.

7. Composition according to claim 3 or 4, which comprises 0.005 to 0.5 wt.-% of a compound according to one of claims 1 to 2, relative to the total mass of the composition.

8. Composition according to one of claims 4 or 7, which comprises at least one mono- or multifunctional (meth)acrylate or a mixture thereof as radically polymerizable monomer.

9. Composition according to one of claims 3 to 8, which comprises
(a) 0.001 to 3 wt.-% of at least one compound of general formula (I),
(b) 1 to 99.9 wt.-% of at least one radically polymerizable monomer,
(c) 0 to 85 wt.-% of at least one filler and
(d) 0 to 70 wt.-% of one or more additives,
in each case relative to the total mass of the composition.

10. Composition according to one of claims 3 to 8, which comprises
(a) 0.001 to 1.0 wt.-% of at least one compound of general formula (I),
(b) 5 to 95 wt.-% of at least one radically polymerizable monomer,
(c) 5 to 80 wt.-% of at least one filler and
(d) 0.1 to 60 wt.-% of one or more additives,
in each case relative to the total mass of the composition.

11. Composition according to one of claims 3 to 8, which comprises
(a) 0.005 to 0.5 wt.-% of at least one compound of general formula (I),
(b) 10 to 90 wt.-% of at least one radically polymerizable monomer,
(c) 10 to 75 wt.-% of at least one filler and
(d) 0.1 to 50 wt.-% of one or more additives,
in each case relative to the total mass of the composition.

12. Composition according to one of claims 3 to 11 for therapeutic use as a dental material.

13. Composition for therapeutic use according to claim 12 as a dental cement, filling composite or veneering material.

14. Non-therapeutic use of a composition according to one of claims 3 to 11 for the production or repair of dental restorations, prostheses, artificial teeth, inlays, onlays, crowns or bridges.

15. Use of a compound according to one of claims 1 to 2 as a photoinitiator.

## Revendications

1. Composé de formule générale (I) : 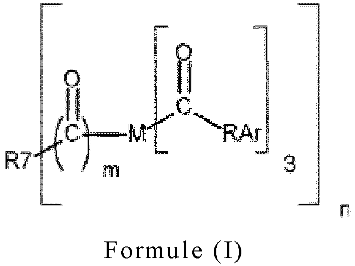 dans laquelle les symboles ont les significations suivantes :
- M représente un atome de germanium ou d'étain,
- RAr représente un groupe de formule 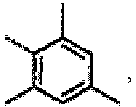
- R⁷ représente un groupe de formule 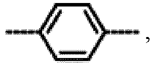
- l'indice n vaut 2,
- et l'indice m vaut 1 ;
ou bien
- M représente un atome de germanium,
- RAr représente un groupe de formule 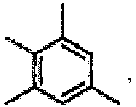
- R⁷ représente un groupe de formule 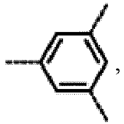
- l'indice n vaut 3,
- et l'indice m vaut 1 ;
ou bien
- M représente un atome de germanium,
- RAr représente un groupe de formule 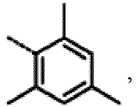
- R⁷ représente un groupe alcanediyle en C₂-C₈,
- l'indice n vaut 2,
- et l'indice m vaut 0.

2. Composé conforme à la revendication 1, dans lequel les symboles de la formule (I) ont les significations suivantes :
- M représente un atome de germanium,
- RAr représente un groupe de formule 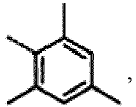
- R⁷ représente un groupe de formule -C₄H₈-,
- l'indice n vaut 2,
- et l'indice m vaut 0.

3. Composition qui contient au moins un composé conforme à l'une des revendications 1 et 2 et au moins un monomère polymérisable.

4. Composition conforme à la revendication 3, qui contient, en tant que monomère polymérisable, un monomère polymérisable par voie radicalaire.

5. Composition conforme à la revendication 3 ou 4, qui contient de 0,001 à 3 % d'un composé conforme à l'une des revendications 1 et 2, en poids rapporté au poids total de la composition.

6. Composition conforme à la revendication 3 ou 4, qui contient de 0,001 à 1 % d'un composé conforme à l'une des revendications 1 et 2, en poids rapporté au poids total de la composition.

7. Composition conforme à la revendication 3 ou 4, qui contient de 0,005 à 0,5 % d'un composé conforme à l'une des revendications 1 et 2, en poids rapporté au poids total de la composition.

8. Composition conforme à l'une des revendications 4 à 7, qui contient, en tant que monomère polymérisable par voie radicalaire, au moins un acrylate ou méthacrylate monofonctionnel ou polyfonctionnel, ou un mélange de tels monomères.

9. Composition conforme à l'une des revendications 3 à 8, qui contient :
a) de 0,001 à 3 % en poids d'au moins un composé de formule générale (I),
b) de 1 à 99,9 % en poids d'au moins un monomère polymérisable par voie radicalaire,
c) de 0 à 85 % en poids d'au moins une charge,
d) et de 0 à 70 % en poids d'un ou de plusieurs adjuvant(s),
poids rapporté, pour chacun, au poids total de la composition.

10. Composition conforme à l'une des revendications 3 à 8, qui contient :
a) de 0,001 à 1,0 % en poids d'au moins un composé de formule générale (I),
b) de 5 à 95 % en poids d'au moins un monomère polymérisable par voie radicalaire,
c) de 5 à 80 % en poids d'au moins une charge,
d) et de 0,1 à 60 % en poids d'un ou de plusieurs adjuvant(s),
poids rapporté, pour chacun, au poids total de la composition.

11. Composition conforme à l'une des revendications 3 à 8, qui contient :
a) de 0,005 à 0,5 % en poids d'au moins un composé de formule générale (I),
b) de 10 à 90 % en poids d'au moins un monomère polymérisable par voie radicalaire,
c) de 10 à 75 % en poids d'au moins une charge,
d) et de 0,1 à 50 % en poids d'un ou de plusieurs adjuvant(s),
poids rapporté, pour chacun, au poids total de la composition.

12. Composition conforme à l'une des revendications 3 à 11, pour utilisation thérapeutique comme matériau dentaire.

13. Composition pour utilisation thérapeutique conforme à la revendication 12, en tant que ciment dentaire, composite de remplissage ou matériau de placage.

14. Utilisation non thérapeutique d'une composition conforme à l'une des revendications 3 à 11 pour la fabrication ou la réparation de restaurations dentaires, de prothèses, de dents artificielles, d'inlays, d'onlays, de couronnes ou de bridges.

15. Utilisation d'un composé conforme à l'une des revendications 1 et 2 en tant que photoamorceur.
